(19) Europäisches Patentamt
European Patent Office
Office européen des brevets

(11) Numéro de publication: 0 283 359 B1

(12) # FASCICULE DE BREVET EUROPEEN

(45) Date de publication du fascicule du brevet: 12.09.90

(51) Int. Cl.[5]: C07C 45/00, C07C 49/00, C08F 4/48, C08F 36/04

(21) Numéro de dépôt: 88400421.9

(22) Date de dépôt: 24.02.88

(54) Procédé de préparation de cétones.

(30) Priorité: 11.03.87 FR 8703307
11.03.87 FR 8703308

(43) Date de publication de la demande: 21.09.88 Bulletin 88/38

(45) Mention de la délivrance du brevet: 12.09.90 Bulletin 90/37

(84) Etats contractants désignés: BE CH DE GB IT LI NL

(56) Documents cités:
SYNTHESIS, no. 1, janvier 1984, pages 37-40; G. FRIOUR et al.: "Organomanganous reagents; IX1. Preparation of various halogenated, alkoxylated, aryloxylated, and arylsulfenylated ketones from correspondingly functionalized carboxylic acid chlorides or anhydrides" an isobutane-isobutylene fraction" enols" trength fibre, e.g. glass fibre" CHEM. 1977, 000
SYNTHESIS, no. 2, février 1977, pages 130-133; G. CAHIEZ et al.: "Reactivity of organomanganese (II) reagents, II. A new, convenient preparation of alkyl, alkenyl, and alkynyl ketones via organomanganese (II) iodides"
TETRAHEDRON LETTERS, no. 36, 1976, pages 3155-3156, Pergamon Press, GB; G. CAHIEZ et al.: "Reactivite des derives

(73) Titulaire: SOCIETE NATIONALE ELF AQUITAINE Société anonyme dite, Tour Elf 2, Place de la Coupole La Défense 6, F-92400 Courbevoie(FR)

(72) Inventeur: Tozzolino, Pierre, Chemin Carrerot Serres-Morlaas, F-64160 Morlaas(FR)
Inventeur: Cahiez, Gérard, 68 rue de Turbigo, F-75003 Paris(FR)
Inventeur: Laboue, Blandine, Tour 44-45 2ème étage 4 Place Jussieu, F-75252 PARIS CEDEX 05(FR)

(74) Mandataire: Kohn, Armand, 5 Avenue Foch, F-92380 Garches(FR)

(56) Documents cités: (suite)
organo-manganeux. I-action sur les chlorures d'acides. Synthèse de cetones"
J.A.C.S., 5 août 1953, pages 3731-3734; W.C. PERCIVAL et al.: "Grignard reactions. XXI.1 The synthesis of aliphatic ketones"

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne un perfectionnement à la préparation de cétones. Elle se rapporte plus spécialement à l'obtention de diverses cétones par l'action d'un composé organo-métallique sur un composé porteur d'un ou de plusieurs groupes carbonyle.

L'utilité des cétones en général est bien connue; parmi les méthodes classiques de leur préparation, celle qui est basée sur l'emploi d'un réactif organo-métallique est fort interessante, parcequ'elle permet d'obtenir des types variés de cétones, utiles notamment dans le domaine pharmaceutique, ainsi que dans celui des arômes et parfums. La voie par composés organo-métalliques a fait l'objet de nombreux travaux, comme entre autres ceux de EBERLE et KAHLE ("Tetrahedron Letters" vol.21, p.2303-2304, 1980) qui décrivent la préparation, à basse température, de cétones porteuses de diverses fonctions, telles qu'halogènes et esters. Un progrès net a été réalisé dans ce domaine par l'emploi d'organo-manganeux qui ont permis d'opérer dans des conditions plus douces, plus commodes. Ainsi, selon G. CAHIEZ ("L'Actualité Chimique"), Septembre 1984, pages 27-28) toutes sortes de cétones ont pu être synthétisées au sein de l'éther ou de tétrahydrofurane, par la réaction $RMnX + R'COZ \rightarrow RCOR'$, R et R' pouvant être des alkyles, alcényles, aryles ou alcynyles, X étant un halogéne et Z un groupe susceptible de se combiner avec -MnX. La réaction peut être réalisée à des températures de 0° à +10°C au lieu de -50° à -70°C qu'exigent les organomagnésiens.

Cependant, sur le plan industriel, où les réactifs R'COZ les plus pratiques sont des chlorures d'acides, l'emploi des organomanganeux présente certaines difficultés. Un premier inconvénient réside en ce que le solvant particulièrement pratique, le THF (tétrahydrofurane), subit l'ouverture de son cycle sous l'action du chlorure d'acide,en présence d'un composé organomanganeux. Un autre désavantage est que, dans un solvant tel qu'éther, la réaction n'a lieu bien que si l'organo-manganeux est un iodure, c.à d. RMnI, ce qui n'est pas acceptable au point de vue économique.

Lorsqu'il s'agit de réaction portant sur des hydrocarbures, par exemple de la réduction d'halogénures vinyliques ou aryliques au moyen de composés organo-métalliques, les écueils signalés ci-dessus peuvent être évités par l'emploi d'un organomagnésien en présence d'un catalyseur constitué par un halogénure de manganèse, en particulier de $MnCl_2$, comme indiqué par CAHIEZ à la page 26 de l'article cité plushaut. La réaction pourrait alors avoir lieu dans du THF ou dans de l'éther et donnerait, selon les cas des rendements d'environ 80 à 95%. Toutefois, l'application de ce procédé catalytique de la réaction de GRIGNARD à la préparation de cétones s'est révélée décevante, à cause de rendements trop bas pour une exploitation industrielle.

La présente invention apporte une solution nouvelle, imprévue, qui permet de produire toutes sortes de cétones par l'action d'un organo-métallique sur un composé organique porteur d'un ou de plusieurs carbonyles, à des températures voisines de l'ambiante, au sein des solvants classiques d'une telle réaction; elle rend possible l'obtention de rendements bien satisfaisants industriellement.

D'après ce procédé on peut dissoudre, dans le solvant approprié, un organo-métallique RMX, où R est un groupe organique, M un métal d'un des groupes I-III et VI-VII de la Classification Périodique des Eléments, et X un halogène, à ajouter à la solution obtenue un sel de Mn en quantité catalytique, et à mélanger cette solution avec une solution, de préférence dans le même solvant, d'un composé porteur d'un ou de plusieurs carbonyles, du type R'COZ où R' est un groupe organique semblable à R, ou différent de R, Z étant tout groupement susceptible de se combiner avec -MX. Le procédé est caractérisé en ce que le sel de Mn, servant de catalyseur, est pris sous la forme d'un sel double de Mn avec un cation alcalin ou ammoniacal.

Ainsi, le catalyseur suivant l'invention est-il constitué par un sel double,manganeux, du type MnX2.nM'Y où X est un anion, par exemple halogène, $1/2\ SO_4$, $1/3\ PO_4$, $BF_4$, $1/3\ BO_3$, $1/2\ SiF_6$, $CH_3COO$-(acétique), de préférence halogène, M' est un cation de métal alcalin ou/et d'ammonium, Y un anion du même type que X mais pas forcément identique,n étant le plus souvent 1 à 4.

Sont d'un emploi très pratique les sels doubles de Mn avec $NH_4$ ou ammonium quaternaire, Na, K ou Li, X et Y étant des halogènes dont - naturellement - Cl est le plus économique. En particulier, le sel double, anhydre, $MnCl_2$. 2LiCl est d'une grande utilité pour la réalisation de l'invention.

La quantité de catalyseur à employer peut varier largement, mais correspond en général à 0,5 à 6 atomes de Mn pour 100 moles de composé organo-métallique RMX, et de préférence à 1-5 atomes Mn/100 moles de ce réactif; les proportions les meilleures se situent entre 2 et 4 atomes de Mn, c. à d. que la quantité de sel double $MnX_2nMY$ est telle qu'il y ait 2 à 4 atomes Mn pour 100 moles de RMX.

Suivant une variante de l'invention, l'activité du catalyseur peut encore être renforcée par l'adjonction d'un sel cuivreux. La proportion pondérale de celui-ci est, de préférence, du même ordre que celle du sel complexe de Mn. Une telle addition est particulièrement utile lorsque le groupe R du composé organométallique, utilisé, est encombré : la présence de Cu permet alors d'augmenter le rendement en cétone, malgré l'encombrement de R.

Grâce au procédé de l'invention, on obtient des bons rendements à des températures allant d'environ –10C à +30°C, le travail étant particulièrement pratique entre –5°C et +10°C pour les monocétones, et entre environ +10°C et 30°C dans le cas des dicétones. Il est possible de tout effectuer dans le solvant économique qu'est le THF, sans aucune altération de ce solvant.

La réaction : $RMX + R'COZ \xrightarrow{MnX_2nM'Y} R\text{-}CO\text{-}R' + MXZ$

ou

$$mRMX + R'(COZ)_m \xrightarrow{MnX_2nM'Y} (RCO)_mR' + mMXZ$$

(m étant surtout 2 ou 3), peut être réalisée avec différents organométalliques RMX, comme indiqué plus haut, et plus particulièrement avec des magnésiens de Grignard, notamment RMgCl.

R peut être tout groupe organique compatible avec le métal M, et surtout un alkyle, alcényle ou alcynyle en $C_1$ à $C_{20}$, un cycloalkyle en $C_5$ ou $C_6$, ou bien un aryle ou alkyl-aryle en $C_6$ à $C_{28}$. Ces radicaux peuvent porter des substituants non réactifs vis-à-vis du métal M.

R′ est un groupe pouvant, comme R, être un alkyle, alcényle, alcynyle, cycloalkyle ou aryle, il peut porter des substituants, notamment des fonctions, ne réagissant pas avec RMX. Par contre Z est un atome ou groupement présen tant de l'affinité vis-à-vis du métal M, par exemple un atome d'halogène ou un carbonyle. Ainsi R′COZ peut être avantageusement un composé tel que R′COCl,

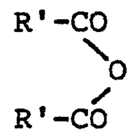

,

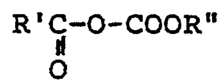

où R″ est un alkyle, etc.

Pour la pratique industrielle les réactifs les plus avantageux sont du type R′COCl, c'est-à-dire les chlorures d'acides.

En ce qui concerne les substituants possibles de R′, ils peuvent être par exemple Cl, Br, -OR, -SR, -COOR, CN, -CO etc. qui permettent l'obtention de cétones fonctionnelles, correspondantes.

Ainsi, le procédé de l'invention permet-il de préparer aisément une grande diversité de cétones saturées, éthyléniques, aromatiques et acétyléniques, éventuellement porteuses d'autres groupes fonctionnels, la production pouvant se faire avec de très bons rendements. La durée de la réaction dépend, bien entendu, de la température, de la nature du composé organométallique et du composé acylant R′COZ, ainsi que de celle du catalyseur particulier, utilisé; elle peut notamment varier entre 0,5 et 10 heures, mais il est généralement possible de régler les conditions opératoires de façon à ce que la réaction soit terminée en environ 1 à 4 heures.

Les excellents résultats, auxquels conduit le procédé de la présente invention, et que l'on peut voir dans les exemples donnés plus loin, sont d'autant plus inattendus que des essais antérieurs de préparation de cétones à partir d'organomanganeux, utilisant le sel complexe $MnCl_2.2LiCl$, se sont révélés fort délicats et impossibles à industrialiser. De tels essais sont décrits dans SYNTHESIS, nº1, Janvier 1984, pages 37-38, par G. FRIOUR et coll. Il est vrai que, contrairement à la présente invention, le sel complexe en question n'a pas été employé comme catalyseur de la réaction d'un magnésien avec un chlorure d'acide, mais comme réactif en proportion stoechiométrique pour la préparation d'un organo-manganeux à partir d'un organo-magnésien. C'est seulement l'organo-manganeux, une fois préparé, que l'on faisait réagir avec un composé R′COZ: on était obligé de travailler en présence d'éther et à basse température, jusqu'à -80°C (page 3 "Method C"); si le THF était employé, sa quantité devait être minimale. Aux températures plus élevées, les rendements baissaient rapidement. Par contre, selon la présente invention, il suffit de quelques % de $MnCl_2.2LiCl$ comme catalyseur, en solution dans du THF avec un composé R′-COZ, pour qu'une addition progressive d'un magnésien RMgX à cette solution produise une cétone RCOR′ avec bon rendement à une température douce, par exemple de 10°C, sans aucun refroidissement préalable.

L'invention est illustrée par les exemples non limitatifs qui suivent.

Mode opératoire général dans les exemples.

A 50 ml de THF anhydre, on ajoute, à la température ambiante, 3 mmoles de $MnCl_2$ sec et 6 mmoles de LiCl sec. Le mélange est alors agité jusqu'à dissolution complète des sels puis additionné de 100 mmoles de RCOCl dans 50 ml de THF. On introduit ensuite 100 mmoles de RMgX à l'aide d'une pompe. La température du milieu réactionnel pendant l'addition de RMgX, ainsi que la durée de cette addition (généralement 0 à +10°C et 30 mn) sont indiquées dans le tableau III. Il est important de maintenir une vive agitation pendant toute la durée de l'introduction de RMgX. Après cette dernière, l'agitation est maintenue 15 à 30 mn à la température ambiante, puis le milieu réactionnel est hydrolysé avec une solution d'HCl dilué. Le produit est ensuite isolé selon les techniques habituelles.

EXEMPLES 1 à 5

L'organo-métallique RMX est ici le chlorure ou le bromure de butyl-magnésium, $C_4H_9MgCl$ (ou Br); un essai avec du butyl-lithium est fait pour comparaison. Le composé à groupe carbonyle, R'COZ, est un dérivé de l'acide octanoïque (caprylique), $C_7H_{15}$-COOH, notamment chlorure (Z = Cl), anhydride ou ester.

Le catalyseur est le sel double $MnCl_2.2LiCl$ employé à raison de 3 moles pour 100 moles de BuMgCl ou BuMgBr.

Au tableau des résultats ci-après on indique le solvant de la solution de butyl-magnésium et celui du milieu réactionnel, auquel cette solution est ajoutée.

Les rendements en cétone $C_4H_9$-CO-$C_7H_{15}$, butyl heptyl cétone, est exprimé par rapport au composé butyl-magnésien de départ.

TABLEAU I

| Exemple n° | RMX/solvant | $C_7H_{15}COZ$ | solvant | Rdt. % |
|---|---|---|---|---|
| 1 | BuMgCl/THF | $C_7H_{15}COCl$ | THF | 87 |
| 2 | BuMgBr/éther | " | " | 82 |
| 3 | BuLi/éther | " | Ether | 52 |
| 4 | BuMgCl/THF | $C_7H_{15}C(=O)$-OCOEt | THF | 75 |
| 5 | BuMgCl/THF | $(C_7H_{15}CO)_2O$ | " | 22 |

Les exemples 1 et 2 montrent, par comparaison avec les autres, que les meilleurs résultats sont obtenus avec RMgCl (ou Br) lorsque le composé R'COZ est un chlorure d'acide.

EXEMPLES 6 à 20

Les matières que l'on fait réagir sont ici le chlorure de butyl magnésium et le chlorure de l'acide octanoïque, dans du tétrahydrofuranne (THF), en vue de la réaction:

$$C_4H_9MgCl + C_7H_{15}COCl \xrightarrow[THF]{} C_4H_9COC_7H_{15} + MgCl_2$$

Comme dans les exemples précédents, les quantités de catalyseur sont exprimées en % molaires du composé organo-métallique, c'est-à-dire en nombre d'atomes de Mn pour 100 moles de $C_4H_9MgCl$.

Les rendements en cétone sont rapportés aux quantités de magnésien mis en oeuvre.

Les résultats sont réunis au Tableau II.

TABLEAU II

| Exemple n° | Nature du catalyseur | Quantité de catalyseur (mole %) | Température (°C) | Durée de l'addition de BuMgCl (en mn) | Rendement (%) |
|---|---|---|---|---|---|
| A/ Influence de la nature du catalyseur | | | | | |
| 6 | $MnCl_2$ | 3% | 0 à +10 | 30 | 60 |
| 7 | $MnCl_4Li_2$ | " | " | " | 87 |
| 8 | $MnBr_4Li_2$ | " | " | " | 84 |
| 9 | $Mn(OOC-CH_3)_2$ | " | " | " | 76 |
| B/ Influence de la quantité de catalyseur | | | | | |
| 10 | $MnCl_4Li_2$ | 1% | 0 à +10 | 30 | 70 |
| 11 | " | 3% | " | " | 87 |
| 12 | " | 5% | " | " | 82 |
| C/ Influence de la vitesse d'addition de BuMgCl | | | | | |
| 13 | $MnCl_4Li_2$ | 3% | 0 à +10 | 15 | 61 |
| 14 | " | " | " | 30 | 87 |
| 15 | " | " | " | 60 | 72 |
| D/ Influence de la température | | | | | |
| 16 | $MnCl_4Li_2$ | 5% | -5 | 30 | 85 |
| 17 | " | " | +10 | " | 82 |
| 18 | " | " | +20 | " | 82 |
| 19 | sans catalyseur | | -5 | " | 36 |
| 20 " | " | | +10 | " | 27 |

Les exemples 7, 11 et 14, faits dans les mêmes conditions que l'exemple 6, montrent l'amélioration considérable du rendement grâce à l'emploi d'un complexe de $MnCl_2$ avec 2 moles de LiCl, au lieu de $MnCl_2$ seul. En effet, le rendement, dans les exemples 7, 11 et 14 s'élève à 87% contre 60% pour l'exemple 6, soit 1,45 fois ce dernier.

Les exemples 19 et 20, où aucun catalyseur n'est utilisé, conduisent à des rendements très bas.

EXEMPLES 21 à 35

Diverses cétones ont été préparées suivant la réaction

$RMgCl + R'COCl \rightarrow RCOR' + MgCl_2$

avec 3 moles de $MnCl_4Li_2$ pour 100 moles RMgCl, en utilisant différents R et R'.

Le Tableau III résume les résultats obtenus.

<u>TABLEAU III</u>

| Exemple n° | R | R' | Température (C°) | Durée d'addition de RMgCl (mn) | Rendement en cétone isolée (%) |
|---|---|---|---|---|---|
| A/Cétones saturées et insaturées | | | | | |
| 21 | Bu | Hept | O à +10 | 30 | 87 |
| 22 | Hept | i-Pr | " | " | 94 |
| 23 | " | t-Bu | " | " | 52 |
| 24 | " | $Me_2C=CH$ | " | " | 79 |
| 25 | Bu | Ph | O | 45 | 85 |
| 26 | i-Pr | Hept | " | 30 | 74 |
| 27 | t-Bu | " | O à +10 | " | 27 |
| 28 | $Me_2C=CH$ a) | " | O | 40 | 73 |
| 29 | Ph | Bu | +25 à +30 | 30 | 70 |
| 30 | i-Pent b) | i-Bu | O à +10 | " | 83 |
| B/Cétones fonctionnelles | | | | | |
| 31 | Bu | $-(CH_2)_3COOEt$ | O à +10 | 30 | 62 |
| 32 | " | $-(CH_2)_4COOEt$ | " | " | 83 |
| 33 | " | $-(CH_2)_3Cl$ | " | " | 58 |
| 34 | " | $-(CH_2)_{10}Br$ | " | " | 71 |
| 35 | " | $-(CH_2)_6CN$ | " | " | 84 |

a/ $Me_2C=CHMgBr$

b/ Dans ce cas la réaction a été réalisée en additionnant i-PentMgBr en solution dans l'éther (1,1N).

Au cours de ce travail on a trouvé qu'avec R' constitué par un phényle (exemple 25) il était préférable de travailler à 0°C et un peu plus longtemps (45mn) que dans les autres cas. Il en fut de même avec un alcényle en R (exemple 28). Par contre, on peut opérer vers 25° à 30°C, lorsque R est un phényle (ex. 29).

L'empêchement stérique joue un rôle qui est visible aux exemples 23 et 27, où le rendement est abaissé du fait de l'emploi en R ou en R' d'un alkyle encombré, le tert. butyle en l'occurrence.

Il est intéressant de constater que la présence d'un atome ou groupe fonctionnel Q dans R' (exemples 31 à 35) n'empêche pas l'obtention d'une cétone munie de cet atome ou groupe Q. Si, dans ce cas, R' est du type $Q-(CH_2)_n$, le rendement est d'autant meilleur que n est plus grand.

Ces résultats permettront à l'homme de l'art d'appliquer les conditions de travail les plus favorables dans chaque cas particulier.

EXEMPLES 36-37

En opérant comme dans les exemples 23 et 27, on a expérimenté l'action de CuCl employé conjointement avec du $MnCl_22LiCl$, chacun à raison de 3 moles pour 100 moles de RMgCl.

Les rendements en cétone sont alors:

| Exemple | R | R' | Rendement % |
|---|---|---|---|
| 23 | Hept | t-Bu | 52 |
| 36 (+CuCl) | " | " | 98 |
| 27 | t-Bu | Hept | 27 |
| 37 (+CuCl) | " | " | 80 |

On peut constater qu'aussi bien pour un R encombré (t-Bu) que pour R' encombré (t-Bu), la présence de CuCl augmente considérablement le rendement en cétone.

Dans les exemples qui suivent on a illustré la préparation de dicétones par le même procédé de l'invention.

Voici le mode opératoire appliqué.

A 500 ml de THF anhydre, on ajoute, à la température ambiante, 4g de $MnCl_2$ sec et 1,5 g de LiCl sec. Le mélange est alors agité, jusqu'à dissolution complète des sels, puis additionné de 1 mole de $R'(COCl)_2$ dans 500 ml de THF. On introduit ensuite, progressivement, 2 moles de RMgX à l'aide d'une pompe. La température du milieu réactionnel, pendant l'addition de RMgX est de 25° à 30°C. Il est important de maintenir une vive agitation pendant toute la durée de l'introduction de RMgX. Après cette dernière, l'agitation est maintenue encore durant 1 heure à la température ambiante, puis le milieu réactionnel est hydrolysé avec une solution d'HCl dilué. Le produit est ensuite isolé selon les techniques habituelles.

EXEMPLE 38

Selon le mode opératoire général décrit plus haut, on fait réagir 203 g (1 mole) de dichlorure d'isophtaloyle dans 500 ml de THF, avec 274 g (2 moles) de chlorure de phénylmagnésium.

L'hydrolyse subséquente est effectuée à l'aide d'une solution diluée d'acide chlorhydrique. Par refroidissement, la dicétone cristallise. On obtient le dibenzoyl-1,3 benzène avec un rendement de 75%. Le produit est recristallisé dans l'éthanol. On obtient des cristaux blancs, dont le point de fusion est de 100°C, qui répondent à la formule:

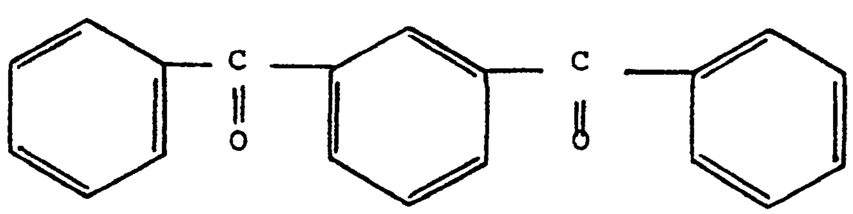

EXEMPLE 39

En remplaçant le chlorure de phénylmagnésium par le chlorure de méthyl-4 phénylmagnésium, on obtient le di(méthyl4 benzoyl)-1,3 benzène avec un rendement de 70%. Le produit est recristallisé dans l'éthanol. On obtient des cristaux blancs qui fondent à 126° et 127°C.

EXEMPLE 40

Le remplacement du dichlorure d'isophtaloyle par du dichlorure d'hexanoyle donne la diphényl-1,6 hexanedione-1,6 avec un rendement de 65%. Après recristallisation, le produit fond à 106°C.

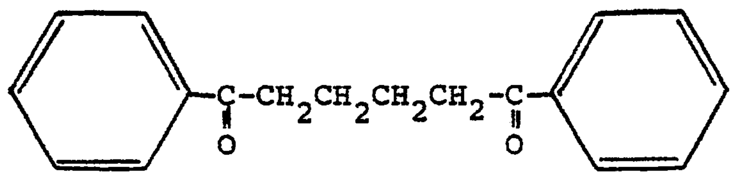

EXEMPLE 41

Dans l'exemple 38, on a remplacé le chlorure de phénylmagnésium par du chlorure de butylmagnésium. On a obtenu le dipentanoyl-1,3 benzène avec un rendement en produit isolé de 75%:

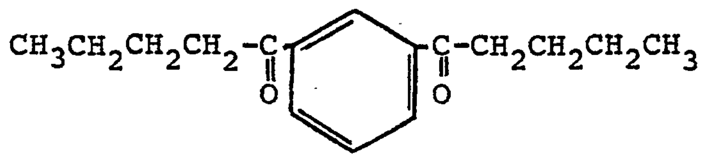

EXEMPLE 42

Application de la dicétone dans l'exemple 38.

Elle est transformée en diol puis en diène. Ces deux réactions peuvent être mises en oeuvre sans isolement de cette dicétone intermédiaire.

Après réaction du chlorure de phénylmagnésium, le milieu réactionnel est refroidi vers -10°C et 27g (1,20 moles) de méthyllithium sont ajoutés. La réaction est poursuivie pendant 2 heures, la température remontant lentement à l'ambiante. On procède alors à l'hydrolyse du milieu avec de l'acide chlorhydrique dilué, à une température comprise entre -10 et 0°C.

Après lavage, le THF est éliminé et le résidu repris par 500 ml de toluène auquel on ajoute 0,2 g d'un catalyseur de type acide, tel que l'acide p-toluènesulfonique, et on porte l'ensemble au reflux pendant 1h30. Le milieu est neutralisé et le toluène éliminé sous pression réduite. Le produit brut de réaction est une huile visqueuse de couleur jaune-orangée. Elle est reprise par l'éthanol bouillant et par refroidissement de la solution on obtient des cristaux incolores de di [(phényl)-éthényl]-1,3 benzène dont le point de fusion est de 46°C.

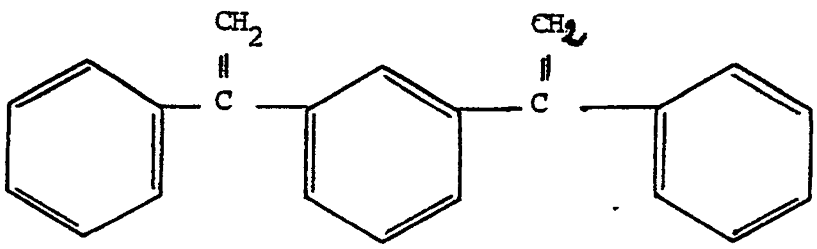

EXEMPLE 43

La dicétone obtenue dans l'exemple 39 est traitée selon le mode opératoire del'exemple 42. On obtient, après recristallisation dans l'éthanol, des cristaux blancs de di [(méthyl-4 phényl)éthényl]-1,3 benzène qui fondent à 66°C. Les deux diènes obtenus dans les exemples 42 et 43 sont transformés, après réaction avec un alkyllithium tel que le sec.butyllithium, en dérivés organolithiens qui sont communément utilisés comme initiateurs dans la polymérisation de diènes, tels que le butadiène ou l'isoprène, dans la configuration 1-4 favorable aux propriétés de l'élastomère qui en résulte.

## Revendications

1. Procédé de préparation de cétones par la réaction d'un composé organo-métallique avec un composé porteur d'un ou de plusieurs carbonyles au sein d'un solvant, en présenced'un sel manganeux comme catalyseur, caractérisé en ce que le sel manganeux est sous la forme d'un sel double de Mn et d'un cation de métal alcalin ou d'ammonium.

2. Procédé suivant la revendication 1, caractérisé en ce que le sel double, servant de catalyseur, est du type $MnX_2.nM'Y$ où X et Y, semblables ou différents, sont des anions, M' est un atome de métal alcalin, $NH_4$ en particulier ammonium quaternaire, Na, K ou Li, et n a une valeur de 1 à 4.

3. Procédé suivant la revendication 2, caractérisé en ce que X et Y sont des halogènes, en particulier Cl ou Br.

4. Procédé suivant la revendication 2, caractérisé en ce que X et Y sont des anions 1/2 $SO_4$, 1/3 $PO_4$, $BF_4$, 1/3 $BO_3$, 1/2 $SiF_6$ ou $CH_3COO$.

5. Procédé suivant une des revendications précédentes, dans lequel le métal du composé organo-métallique appartient à un des groupes I-III ou VI-VII de la Classification Périodique des Eléments, particulièrement magnésium.

6. Procédé suivant une des revendications précédentes, caractérisé en ce qu'une solution de composé organo-métallique est versée progressivement dans une solution de dit composé porteur d'un ou de plusieurs carbonyles, renfermant le sel double servant de catalyseur.

7. Procédé suivant une des revendications précédentes, caractérisé en ce que, dans le composé organo-métallique employé, RMX, M est le Mg, X est Cl ou Br, R est un radical alkyle, alcényle ou alcynyle en $C_1$ à $C_{20}$, cycloalkyle en $C_5$ ou $C_6$, ou bien un aryle ou alkyl-aryle en $C_6$ à $C_{28}$, pouvant porter des substituants non réactifs vis-à-vis du métal M.

8. Procédé suivant une des revendications précédentes, caractérisé en ce que, dans le composé porteur d'un ou de plusieurs carbonyles, R'COZ, R' est un radical alkyle, alcényle ou alcynyle en $C_1$ à $C_{20}$, cycloalkyle en $C_5$ ou $C_6$, ou bien un aryle ou alkyl-aryle en $C_6$ à $C_{28}$, pouvant porter des substituants non réactifs vis-à-vis du métal M, Z étant un atome ou groupement présentant de l'affinité vis-à-vis de M.

9. Procédé suivant une des revendications précédentes, caractérisé en ce que le catalyseur est additionné d'un sel cuivreux.

10. Procédé suivant une des revendications précédentes, caractérisé en ce que le solvant est le tétrahydrofurane.

11. Procédé suivant une des revendications précédentes, caractérisé en ce que la quantité de sel double de Mn, servant de catalyseur, est telle qu'il y ait 0,5 à 6 - et de préférence 2 à 4 - atomes de Mn pour 100 moles de composé organo-métallique.

12. Procédé suivant une des revendications précédentes, caractérisé en ce que la réaction est conduite à une température comprise entre -10° et +30°C.

13. Procédé suivant une des revendications précédentes, appliqué à la préparation de dicétones utilisées en tant qu'initiateurs de l'addition -1,4 dans la polymérisation du butadiène ou de l'isoprène.

14. Application suivant la revendication 13, qui comprend la réaction de la dicétone avec du méthyl lithium, suivie d'une hydrolyse acide, puis chauffage à reflux, dans un solvant, avec un catalyseur acide, caractérisé en ce que cette transformation a lieu dans le milieu même de préparation de la cétone.

## Patentansprüche

1. Verfahren zur Herstellung von Ketonen durch Reaktion einer metallorganischen Verbindung mit einer Verbindung, die ein oder mehrere Carbonyle aufweist, in einem Lösungsmittel in Gegenwart eines Mangansalzes als Katalysator, dadurch gekennzeichnet, daß das Mangansalz in Form eines Doppelsalzes von Mn mit einem Alkalimetall- oder Ammoniumkation vorliegt.

2. Verfahren nach Anspruch 1, dadurch gekennzeichnet, daß das Doppelsalz, das als Katalysator dient, vom Typ $MnX_2.nM'Y$ ist, worin X und Y, gleich oder verschieden, Anionen sind, M' ein Alkalimetallatom oder $NH_4$ ist, insbesondere quaternäres Ammonium, Na, K oder Li, und n einen Wert von 1 bis 4 hat.

3. Verfahren nach Anspruch 2, dadurch gekennzeichnet, dass X und Y Halogene sind, insbesondere Cl oder Br.

4. Verfahren nach Anspruch 2, dadurch gekennzeichnet, dass X und Y die Anionen 1/2 $SO_4$, 1/3 $PO_4$, $BF_4$, 1/3 $BO_3$, 1/2 $SiF_6$ oder $CH_3COO$ sind.

5. Verfahren nach einem der vorstehenden Ansprüche, worin das Metall der metallorganischen Verbindung einer der Gruppen I–II oder VI–VII des Periodensystems der Elemente angehört und insbesondere Magnesium ist.

6. Verfahren nach einem der vorstehenden Ansprüche, dadurch gekennzeichnet, dass eine der metallorganischen Verbindung fortschreitend in eine Lösung besagter, ein oder mehrere Carbonyle aufweisenden Verbindung, die das als Katalysator dienende Doppelsalz enthält, gegossen wird.

7. Verfahren nach einem der vorstehenden Ansprüche, dadurch gekennzeichnet, dass in der verwandten metallorganischen Verbindung RMX M Mg ist, X Cl oder Br ist, R ein Alkyl-, Alkenyl- oder Alkinylrest von $C_1$ bis $C_{20}$, ein Cycloalkylrest von $C_5$ oder $C_6$ oder auch ein Aryl- oder Alkylarylrest von $C_6$ bis $C_{28}$ ist, der gegenüber dem Metall M unreaktive Substituenten aufweisen kann.

8. Verfahren nach einem der vorstehenden Ansprüche, dadurch gekennzeichnet, dass in der ein oder mehrere Carbonyle aufweisenden Verbindung R'COZ R' ein Alkyl-, Alkenyl- oder Alkinylrest von $C_1$ bis $C_{20}$, ein Cycloalkylrest von $C_5$ oder $C_6$ oder auch ein Aryl- oder Alkylarylrest von $C_6$ bis $C_{28}$ ist, der gegenüber dem Metall M unreaktive Substituenten aufweisen kann, wobei Z ein Atom oder eine Gruppe ist, die eine Affinität gegenüber M zeigt.

9. Verfahren nach einem der vorstehenden Ansprüche, dadurch gekennzeichnet, dass dem Katalysator ein Kupfersalz zugesetzt wird.

10. Verfahren nach einem der vorstehenden Ansprüche, dadurch gekennzeichnet, dass das Lösungsmittel Tetrahydrofuran ist.

11. Verfahren nach einem der vorstehenden Ansprüche, dadurch gekennzeichnet, dass die Menge des als Katalysator dienenden Doppelsalzes von Mn so ist, dass sie 0,5 bis 6 - und vorzugsweise 2 bis 4 - Atome Mn auf 100 Mole der metallorganischen Verbindung aufweist.

12. Verfahren nach einem der vorstehenen Ansprüche, dadurch gekennzeichnet, dass die Reaktion bei einer Temperatur zwischen -10° und +30°C durchgeführt wird.

13. Verfahren nach einem der vorstehenden Ansprüche, angewandt auf die Herstellung von Diketonen, soweit sie als Initiatoren bei der 1,4-Addition bei der Polymerisation von Butadien und Isopren verwandt werden.

14. Verwendung nach Anspruch 13, welche die Reaktion des Diketons mit Methyllithium, gefolgt von der sauren Hydrolyse, danach Erhitzen unter Rückfluss in einem Lösungsmittel mit einem sauren Katalysator umfasst, dadurch gekennzeichnet, dass diese Umwandlung im gleichen Milieu stattfindet, wie die Herstellung des Ketons.

## Claims

1. Process of preparation of ketones by the reaction of an organometallic compound with a compound carrying one or more carbonyls in a solvent, in the presence of a manganous salt as catalyst, characterised in that the manganous salt is in the form of a double salt of Mn and a cation of an alkali metal or ammonium.

2. Process according to claim 1, characterised in that the double salt serving as the catalyst is of the type $MnX_2.nM'Y$ where X and Y are the same or different and are anions, M, is an atom of an alkali metal, $NH_4$, in particular a quatern ary ammonium, Na, K or Li and n has a value from 1 to 4.

3. Process according to claim 2, characterised in that X and Y are halogens, in particular Cl or Br.

4. Process according to claim 2, characterised in that X and Y are the anions 1/2 $SO_4$, 1/3 $PO_4$, $BF_4$, 1/3 $BO_3$, 1/2 $SiF_6$ or $CH_3COO$.

5. Process according to any of the preceding claims, in which the metal of the organo-metallic compound belongs to one of the groups I–III or VI–VII of the Period Classification of the elements, particularly magnesium.

6. Process according to any of the preceding claims, characterised in that a solution of the organometallic compound is poured progressively into a solution of the compound carrying one or more carbonyls, containing the double salt serving as the catalyst.

7. Process according to any of the preceding claims, characterised in that in the organo-metallic compound employed, RMX, M is Mg, X is Cl or Br, R is a $C_1$ to $C_{20}$ alkyl, alkenyl or alkynyl group, a $C_5$ or $C_6$ cycloalkyl or a $C_6$ to $C_{28}$ aryl or alkyl-aryl, which can carry substituents which are not reactive with the metal M.

8. Process according to any of the preceding claims, characterised in that in the compound carrying one or more carbonyl groups, R'COZ, R' is a $C_1$ to $C_{20}$ alkyl, alkenyl or alkynyl group, a $C_5$ or a $C_6$ cycloalkyl or $C_6$ to $C_{28}$ aryl or alkyl-aryl which can carry substituents not reactive with the metal M, Z being an atom or group having affinity with M.

9. Process according to any of the preceding claims, characterised in that a cuprous salt is added to the catalyst.

10. Process according to any of the preceding claims, characterised in that the solvent is tetrahydrofuran.

11. Process according to any of the preceding claims, characterised in that the quantity of the double Mn salt serving as the catalyst is such that there are 0.5 to 6 and preferably 2 to 4 atoms of Mn per 100 moles of the organo-metallic compound.

12. Process according to any of the preceding claims, characterised in that the reaction is carried out at a temperature in the range from -10° to +30°C.

13. Process according to any of the preceding claims, employed for the preparation of diketones utilised as initiators for 1,4 addition in the polymerisation of butadiene or isoprene.

14. Use according to claim 13, which comprises the reaction of the diketone with methyllithium followed by acid hydrolysis then heating under reflux in a solvent with an acid catalyst, characterised in that this conversion takes place in the medium used for preparation of the ketone.